# EUROPEAN PATENT SPECIFICATION

(11) **EP 4 166 096 B1**
(45) Date of publication and mention of the grant of the patent: **18.12.2024**
(21) Application number: 21825811.9
(22) Date of filing: 15.06.2021
(51) Int. Cl.: A61M 13/00

(54) **CONSTANT-PRESSURE VARIABLE-FLOW INSUFFLATOR**
INSUFFLATOR MIT KONSTANTEM DRUCK UND VARIABLEM DURCHFLUSS
INSUFFLATEUR À DÉBIT VARIABLE À PRESSION CONSTANTE

(30) Priority: 15.06.2020 CN 202010540723
(43) Date of publication of application: 19.04.2023
(73) Proprietor: Nanjing Tuge Healthcare Co., Ltd., Nanjing, Jiangsu 211100 (CN)
(72) Inventor: HUANG, Xinjun, Nanjing, Jiangsu 211100 (CN); ZHAO, Weiwei, Nanjing, Jiangsu 211100 (CN); XU, Jianglin, Nanjing, Jiangsu 211100 (CN)
(74) Representative: Schrell, Andreas
(86) International application number: PCT/CN2021/100203
(87) International publication number: WO 2021/254352

(56) References cited:
- WO-A1-2019/130119
- WO-A1-2019/130119
- WO-A1-2020/115835
- CN-A- 105 011 973
- CN-A- 105 011 973
- CN-A- 105 012 011
- CN-A- 108 634 999
- CN-A- 111 685 831
- CN-Y- 2 927 982
- CN-Y- 2 927 982
- JP-A- 2012 205 748
- US-A1- 2005 234 391
- US-A1- 2014 236 074
- US-A1- 2019 307 972
- US-B2- 8 172 787

## Description

### TECHNICAL FIELD

The present application relates to the technical field of insufflator equipment, and in particular, to a constant-pressure variable-flow insufflator.

### BACKGROUND

As a standard configuration of laparoscopic surgery, an insufflator achieves an effect of inflating an abdominal cavity in hepatobiliary surgery, gastrointestinal surgery, etc., so as to obtain a good surgical operation space. The insufflator is an important equipment for the endoscopic surgery. It inflates and bulges a surgical site or cavity, which facilitates the observation and diagnosis of a doctor. Since the equipment belongs to micro-pressure and micro-flow precision control equipment (the working pressure is generally 0-30 mmHg, and the maximum gas delivery rate is generally less than 40 LPM), there are problems such as equipment preparation difficulties, high risk of equipment use, etc. The out of control of pressure of gas will have a serious impact on a surgery, and may also endanger the life safety of a patient.

United States Patent Application Publication No. US 2014/0236074 A1 discloses that the pneumatic system for intussusception treatment, i.e., invagination of a segment of the intestine into an adjacent segment, includes a pressurized gas supply connected to a series of filters, valves, regulators, and sensors connected to a rectal insertion tube to introduce gas at moderate pressure into the intestine of the patient. A computerized control and monitoring subsystem is included. The system preferably includes a heating system to warm the gas as desired. The system also preferably includes an exhaust portion to relieve internal intestinal pressure as required or desired. The exhaust portion of the system preferably includes a filter to absorb undesirable fecal odors that accompany the exhausted gas. At least the rectal insertion tube and the odor filter may be separable from the remainder of the system for convenient disposal. An alarm may be provided to alert the doctor or medical professional of conditions other than normal.

United States Patent Application Publication No. US 2019/0307972 A1 discloses that an insufflation device for use in medical engineering comprising an insufflator for gas supply with a gas source, a controller unit, an insufflation line and a separate desufflation line, wherein the desufflation line is connected to a suction pump, wherein the insufflation line and the desufflation line have one pressure sensor and one volume flow sensor each, wherein the insufflator comprises a device for controlled ventilation of the gas present in the patient, wherein the controller unit controls the power of the suction pump as a function of the pressure measurement of the pressure sensors, wherein the controller unit contains an activation blocking system that prevents an activation of the suction pump, when the pressure measured by means of the pressure sensor in the insufflation line is lower than a preset threshold value, wherein the activation blocking system further prevents an activation of the suction pump, when the pressure measured by means of the pressure sensor in the insufflation line and the pressure measured by means of the pressure sensor in the ventilation line are not identical.

The existing insufflator adopts a periodic control technology of supplying gas and measuring pressure at different times. The error range of a pressure regulation interval is large, and the gas supply flow changes nonlinearly. Under the action of a switching valve, a pressure pulse will be generated. The peak value of the pressure pulse often exceeds a set pressure value. Under a dynamic condition that the leakage gas flow changes greatly, the maximum output gas pressure of the insufflator can even reach 40 mmHg, which is far beyond a clinical safety threshold value. Meanwhile, pulsed gas supply easily causes a pressure impact on the abdominal cavity of the patient. In addition, an instable equipment regulation and control period, poor pressure control precision, etc. are also caused due to the uncertainty of operating time. Therefore, it is necessary to develop a simpler insufflator to realize a constant-pressure variable-flow process.

### SUMMARY

Based on this, it is necessary to provide a constant-pressure variable-flow insufflator.

In order to achieve the abovementioned objective, the present invention provides a constant-pressure variable-flow insufflator as claimed in the appending claims:

A constant-pressure variable-flow insufflator includes housing. An insufflator gas inlet, an insufflator gas outlet, a smoke removal gas inlet, and a smoke removal gas outlet are respectively provided on an outer side of the housing. The insufflator gas inlet is connected to an external gas source through a pressure relief valve A. The insufflator gas outlet is connectable to an abdominal cavity of a patient through a filter A. The smoke removal gas inlet is connectable to the abdominal cavity of the patient through a filter B. The smoke removal gas outlet is communicated with a vacuum extractor. A control module, a pneumatic module, a temperature detection module, a safety module, a temperature control module, and a smoke removal module are arranged in the housing. The control module is in signal connection with each of the pneumatic module, the temperature detection module, the safety module, the temperature control module, and the smoke removal module for driving a system and performing digital signal processing on collected data. The pneumatic module is configured for controlling pressure, temperature, and flow of gas delivered by the external gas source. The temperature detection module is configured for detecting the temperature of the gas delivered by the external gas source. The safety module is configured for detecting a temperature of the control module and the pneumatic module. The temperature control module is configured for reducing noise while cooling an interior of a chassis. The smoke removal module is configured for exhausting smoke generated in the abdominal cavity of the patient.

The constant-pressure variable-flow insufflator according to the present invention further include that:

The abovementioned pneumatic module includes a filter C, a pressure sensor A, a heating pressure relief valve, a safety valve, a pressure sensor B, a proportional valve module, a flow sensor, and a switching valve module. One end of the filter C is connected to the insufflator gas inlet through a pipeline, and another end of the filter C 31 is connected to one end of the heating pressure relief valve through the pipeline. Another end of the heating pressure relief valve is connected to one end of the safety valve through the pipeline. Another end of the safety valve is connected to the proportional valve module through the pipeline. One end of the proportional valve module is connected to one end of the flow sensor. Another end of the flow sensor is connected to one end of the switching valve module through the pipeline. Another end of the switching valve module is connected to the insufflator gas outlet. The flow sensor is in signal connection with the control module. The pressure sensor A is arranged on the pipeline between the filter C and the heating pressure relief valve. The pressure sensor A is in signal connection with the control module. The pressure sensor B is arranged on the pipeline between the safety valve and the proportional valve module. The pressure sensor B is in signal connection with the control module.

In a preferred embodiment of the present invention, the abovementioned proportional valve module includes a first gas path and a second gas path connected in parallel. The first gas path and the second gas path are not opened at a same time. The first gas path consists of a pressure relief valve C, a pressure sensor C, and a proportional valve C. The second gas path consists of a pressure relief valve D, a pressure sensor D, and a proportional valve D. One end of the pressure relief valve C is connected to the pressure sensor B through the pipeline. Another end of the pressure relief valve C is connected to the proportional valve C through the pipeline. Another end of the proportional valve C is connected to the flow sensor through the pipeline. The pressure sensor C is arranged on the pipeline between the pressure relief valve C and the proportional valve C. The pressure sensor C is in signal connection with the control module. One end of the pressure relief valve D is connected to the pressure sensor B through the pipeline. Another end of the pressure relief valve D is connected to one end of the proportional valve D through the pipeline. Another end of the proportional valve D is connected to the flow sensor through the pipeline. The pressure sensor D is arranged on the pipeline between the pressure relief valve D and the proportional valve D. The pressure sensor D is in signal connection with the control module.

In a further preferred embodiment of the present invention, the abovementioned switching valve module includes a switching valve A, a switching valve B, a pressure sensor E, and a pressure sensor F. The switching valve A and the switching valve B are not opened or closed at a same time. One end of the switching valve A is connected to the flow sensor, and another end of the switching valve A is connected to the insufflator gas outlet. The pressure sensor E and the pressure sensor F are arranged on the pipeline between the switching valve A and the insufflator gas outlet. The pressure sensor E is connected in series with the pressure sensor F. One end of the pressure sensor E is connected to the switching valve B through the pipeline.

In a preferred embodiment of the present invention, the abovementioned temperature detection module includes a temperature sensor A and a temperature sensor B. The temperature sensor A is arranged on the pipeline between the filter C and the pressure sensor A. The temperature sensor A is in signal connection with the control module. The temperature sensor B is arranged on the pipeline between the pressure sensor F and the insufflator gas outlet. The temperature sensor B is in signal connection with the control module.

According to the present invention, the abovementioned safety module includes a temperature sensor C and a temperature sensor D. The temperature sensor C is arranged on an outer side of the heating pressure relief valve. The temperature sensor C is in signal connection with the control module for detecting a temperature of the heating pressure relief valve. The temperature sensor D is mounted on the control module, and is in direct signal connection with the control module for detecting a temperature of the control module.

The abovementioned temperature control module preferably includes a fan and a temperature sensor E. The fan is mounted at a bottom of the control module. The control module is connected to the fan through a circuit. The temperature sensor E is mounted on the control module, and is in direct signal connection with the control module. The control module receives a temperature input by the temperature sensor E, and controls the fan to start to cool an interior of the housing.

In a preferred embodiment of the present invention, the abovementioned smoke removal module includes a switching valve C, a filter D, a particle detector A, a particle detector B, and a speed regulating gas pump. One end of the switching valve C is connected to the smoke removal gas inlet through the pipeline, and another end of the switching valve C is connected to the filter D. Another end of the filter D is connected to one end of the speed regulating gas pump through the pipeline. The particle detector A and the particle detector B are connected to the pipeline between the filter D and the speed regulating gas pump in sequence. Both the particle detector A and the particle detector B are in signal connection with the control module. The switching valve C is in signal connection with the control module. Another end of the speed regulating gas pump is connected to the smoke removal gas outlet through the pipeline.

The abovementioned particle detector A is preferably a PM10 detector; and the particle detector D is a PM2.5 detector.

In a further preferred embodiment of the present invention, a touch screen is also arranged in the abovementioned housing. The touch screen is in signal connection with the control module for displaying data output by various modules inside the insufflator.

Compared with the prior art, the present invention has the following beneficial effects that:

1. According to the constant-pressure variable-flow insufflator of the present application, constant-pressure variable-flow control of gas is realized by improving a conventional gas inlet mode and combining the heating pressure relief valve, the safety valve, the proportional valve module, the flow sensor, the switching valve module, etc., so that the error range of the pressure regulation interval is small, and the gas supply flow changes stably and uniformly. For medical staff, stable gas flow output also voids the inconvenience caused by discontinuously regulating equipment in a surgery process. The constant-pressure variable-flow insufflator has high precision of pressure control, and convenience in operation.

2. The constant-pressure variable-flow insufflator is also provided with the smoke removal module. The smoke caused by the working of an intra-abdominal electro surgical product in a surgery process can be eliminated in time by means of the linkage between the smoke removal module and the pneumatic module. The constant-pressure variable-flow insufflator has a simple structure, a clear operation principle, and convenience in function realization, and meets new clinical requirements for an insufflator.

### BRIEF DESCRIPTION OF THE DRAWINGS

To describe the technical solutions in the embodiments of the present invention or in the prior art more clearly, the following briefly describes the accompanying drawings required for describing the embodiments. Apparently, the accompanying drawings in the following description show merely some embodiments of the present invention, and those of ordinary skill in the art may still derive other drawings from these accompanying drawings without creative efforts.
FIG. 1 is a connection block diagram of a control principle of an insufflator of the present invention;
FIG. 2 is a schematic diagram of specific connections between modules of the insufflator of the present invention;
FIG. 3 is a schematic diagram of compositions of a proportional valve module of the present invention; and
FIG. 4 is a schematic diagram of compositions of a switching valve module of the present invention.

### Reference signs in the accompanying drawings:

1-housing, 2-control module, 3-pneumatic module, 4-temperature detection module, 5-safety module, 6-temperature control module, 7-smoke removal module, 8-touch screen, 11-insufflator gas inlet, 12-insufflator gas outlet, 13-smoke removal gas inlet, 14-smoke removal gas outlet, 101-pressure relief valve A, 102-external gas source, 103-filter A, 104-filter B, 105-vacuum extractor, 31-filter C, 32-pressure sensor A, 33-heating pressure relief valve, 34-safety valve, 35-pressure sensor B, 36-proportional valve module, 37-flow sensor, 38-switching valve module, 41-temperature sensor A, 42-temperature sensor B, 51-temperature sensor C, 52-temperature sensor D, 61-fan, 62-temperature sensor E, 71-switching valve C, 72-filter D, 73-particle detector A, 74-particle detector B, 75-speed regulating gas pump, 361-pressure relief valve C, 362-pressure sensor C, 363-proportional valve C, 364-pressure relief valve D, 365-pressure sensor D, 366-proportional valve D, 381-switching valve A, 382-switching valve B, 383-pressure sensor E, and 384-pressure sensor F.

### DETAILED DESCRIPTION OF THE EMBODIMENTS

Technical solutions in the embodiments of the present invention will be clearly and completely described below with reference to the accompanying drawings of the embodiments of the present invention. Apparently, the described embodiments are only part rather than all of the embodiments of the present invention. The scope of the present invention is defined by the independent claim. All other embodiments described in the present disclosure which are not falling within the scope of the claims do not belong to the present invention.

In order to make the abovementioned objective, features, and advantages of the present invention more apparent and more comprehensible, the present invention is further described in detail below with reference to the accompanying drawings and specific implementations.

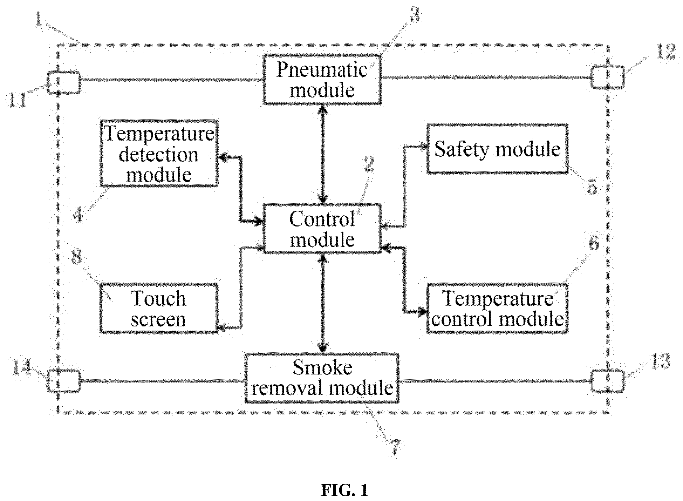

Referring to FIG. 1, a constant-pressure variable-flow insufflator includes a housing 1. An insufflator gas inlet 11, an insufflator gas outlet 12, a smoke removal gas inlet 13, and a smoke removal gas outlet 14 are respectively provided on the outer side of the housing 1. The insufflator gas inlet 11 is connected to an external gas source 102 through a pressure relief valve A 101. The external gas source is preferably carbon dioxide. The insufflator gas outlet 12 is connected to the abdominal cavity of a patient through a filter A 103. The smoke removal gas inlet 13 is connected to the abdominal cavity of the patient through a filter B 104. The smoke removal gas outlet 14 is communicated with a vacuum extractor 105. Preferably, the insufflator gas inlet 11 and the smoke removal gas outlet 14 are located on the same side of the housing; and the insufflator gas outlet 12 and the smoke removal gas inlet 13 are located on the same side of the housing, which is beneficial to ensuring the continuity of the overall operation of the insufflator. A control module 2, a pneumatic module 3, a temperature detection module 4, a safety module 5, a temperature control module 6, and a smoke removal module 7 are arranged in the housing 1. The control module 2 is in signal connection with each of the pneumatic module 3, the temperature detection module 4, the safety module 5, the temperature control module 6, and the smoke removal module 7 for driving a system and performing digital signal processing on the collected data. The pneumatic module 3 is used for controlling the pressure, temperature, and flow of the gas delivered by the external gas source. The temperature detection module 4 is used for detecting the temperature of the gas delivered by the external gas source. The safety module 5 is used for detecting the temperature of the control module and the pneumatic module. The temperature control module 6 is used for reducing noise while cooling the interior of a chassis. The smoke removal module 7 is used for exhausting the smoke generated in the abdominal cavity of the patient.

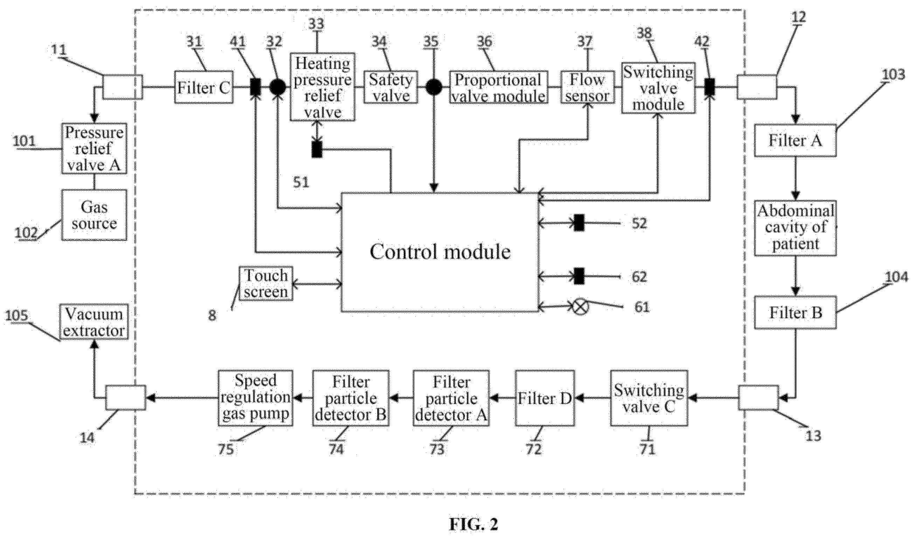

Referring to FIG. 2, in the present embodiment, the pneumatic module 3 includes a filter C 31, a pressure sensor A 32, a heating pressure relief valve 33, a safety valve 34, a pressure sensor B 35, a proportional valve module 36, a flow sensor 37, and a switching valve module 38. One end of the filter C 31 is connected to the insufflator gas inlet 11 through a pipeline, and the other end of the filter C 31 is connected to one end of the heating pressure relief valve 33 through the pipeline. The other end of the heating pressure relief valve 33 is connected to one end of the safety valve 34 through the pipeline. The other end of the safety valve 34 is connected to the proportional valve module 36 through the pipeline. One end of the proportional valve module 36 is connected to one end of the flow sensor 37. The other end of the flow sensor 37 is connected to one end of the switching valve module 38 through the pipeline. The other end of the switching valve module 38 is connected to the insufflator gas outlet 12. The flow sensor 37 is in signal connection with the control module 2. A pressure sensor A 32 is arranged on the pipeline between the filter C 31 and the heating pressure relief valve 33. The pressure sensor A 32 is in signal connection with the control module 2. A pressure sensor B 35 is arranged on the pipeline between the safety valve 34 and the proportional valve module 36. The pressure sensor B 35 is in signal connection with the control module 2. The heating pressure relief valve 33 can be used for heating and reducing primarily pressure of the gas. The safety valve can be used for ensuring that the gas is in a safe state during primary pressure reducing. The proportional valve module 36 may automatically regulate an amount of the output gas, and realize stable output of the gas according to a built-in algorithm. The flow sensor 37 is used for controlling the gas flow output to the abdominal cavity of the patient in a state of ensuring a constant pressure.

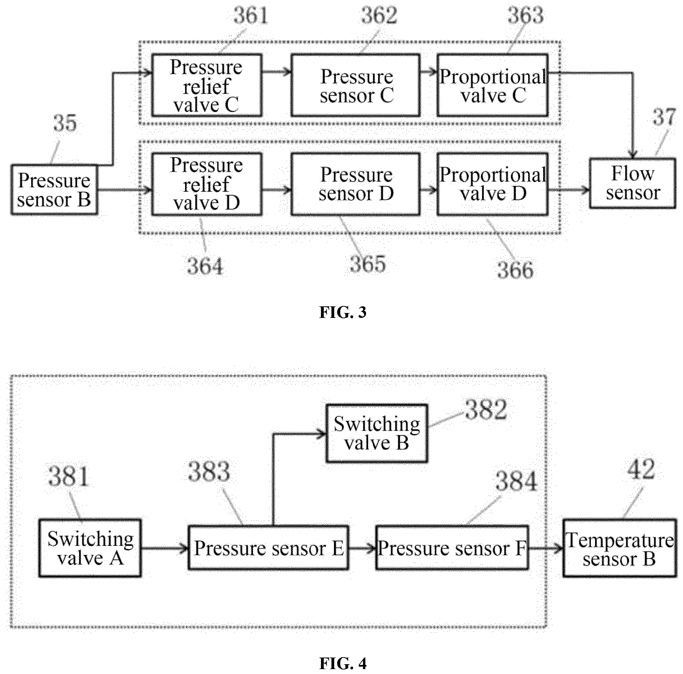

Referring to FIG. 3, in the present embodiment, the proportional valve module 36 includes a first gas path and a second gas path connected in parallel. The first gas path and the second gas path are not opened at the same time. The first gas path consists of a pressure relief valve C 361, a pressure sensor C 362, and a proportional valve C 363. The second gas path consists of a pressure relief valve D 364, a pressure sensor D 365, and a proportional valve D 366. One end of the pressure relief valve C 361 is connected to the pressure sensor B 35 through the pipeline. The other end of the pressure relief valve C 361 is connected to the proportional valve C 363 through the pipeline. The other end of the proportional valve C 363 is connected to the flow sensor 37 through the pipeline. The pressure sensor C 362 is arranged on the pipeline between the pressure relief valve C 361 and the proportional valve C 363. The pressure sensor C 362 is in signal connection with the control module 2. One end of the pressure relief valve D 364 is connected to the pressure sensor B 35 through the pipeline. The other end of the pressure relief valve D 364 is connected to the proportional valve D 366 through the pipeline. The other end of the proportional valve D 366 is connected to the flow sensor 37 through the pipeline. The pressure sensor D 365 is arranged on the pipeline between the pressure relief valve D 364 and the proportional valve D 366. The pressure sensor D 365 is in signal connection with the control module 2. In an actual operation process, there is a requirement on the pressure of the gas entering the abdominal cavity of the patient, which is generally 10 to 15 mmHg, so one pressure sensor is needed to be arranged in any gas path in the proportional valve module. For example, when the first gas path is opened, the pressure relief valve C361 performs secondary pressure reducing on the gas, and the pressure of the gas is detected by the pressure sensor C362. When a detection value detected by the pressure sensor C 362 is consistent with the preset pressure value of the gas when entering the abdominal cavity of the patient, the gas is directly output through a rear-mounted proportional valve C363. When the detected detection value is too high or too low, the gas is regulated through the proportional valve C363, so as to realize stable output of the gas. In the present embodiment, the first gas path can obtain relatively accurate high flow output, and the second gas path can obtain accurate low flow output. The two gas path can correspond to different control ranges, and can be switched with each other automatically, so as to meet the requirements in various practical applications.

Referring to FIG. 4, in the present embodiment, the switching valve module 381 includes a switching valve A 381, a switching valve B 382, a pressure sensor E 383, and a pressure sensor F 384. The switching valve A 381 and the switching valve B 382 are not opened or closed at the same time. One end of the switching valve A 381 is connected to the flow sensor 37, and the other end of the switching valve A 381 is connected to the insufflator gas outlet 12. The pressure sensor E 383 and the pressure sensor F 384 are arranged on the pipeline between the switching valve A 381 and the insufflator gas outlet 12. The pressure sensor E 383 is connected in series with the pressure sensor F 384. One end of the pressure sensor E 383 is connected to the switching valve B 382 through the pipeline. When a process of gas supplying is performed, the switching valve A 381 is opened, and the switching valve B 382 is closed, the gas passes through the switching valve A 381 and finally enters the abdominal cavity of the patient. When a process of gas exhausting is performed, the switching valve B 382 is opened, the switching valve A 381 is closed, and the gas is exhausted into the atmosphere through the switching valve B 382. The pressure sensor C383 and the pressure sensor D384 ensure secondary measurement of the gas pressure of the same path. If a deviation is greater than a set value, the system will give an alarm automatically. If the deviation is within a range, an average value is brought into an algorithm for operation, which ensures the safety of the system.

In the present embodiment, the temperature detection module 4 includes a temperature sensor A 41 and a temperature sensor B 42. The temperature sensor A 41 is arranged on the pipeline between the filter C 31 and the pressure sensor A 32. The temperature sensor A 41 is in signal connection with the control module 2. The temperature sensor B 42 is arranged on the pipeline between the pressure sensor F 384 and the insufflator gas outlet 12. The temperature sensor B 42 is in signal connection with the control module 2. The temperature sensor A 41 can detect the temperature of the gas just entering the pneumatic module, so that various valves in the subsequent pneumatic module can be prevented from being damaged in a gas delivering process. The temperature sensor B 42 can detect the temperature of the gas before entering the abdominal cavity of the patient, which avoids the damage to the body of the patient since the temperature of the gas is too high.

In the present embodiment, the safety module 5 includes a temperature sensor C 51 and a temperature sensor D 52. The temperature sensor C 51 is arranged on the outer side of the heating pressure relief valve 33. The temperature sensor C 51 is in signal connection with the control module 2 for detecting the temperature of the heating pressure relief valve 33. The heating pressure relief valve 33 is a core part of the pneumatic module, and whether it can operate stably will directly affect the overall ventilation effect, so the temperature of the heating pressure relief valve 33 during operating can be directly monitored. The temperature sensor D 52 is mounted on the control module 2, and is in direct signal connection with the control module 2 for detecting the temperature of the control module 2. Thus, a real-time temperature of a main control module during operating can be directly detected.

In the present embodiment, the temperature control module 6 includes a fan 61 and a temperature sensor E 62. The fan 61 is mounted at the bottom of the control module 2. The control module 2 is connected to the fan 61 through a circuit. The temperature sensor E 62 is mounted inside the housing 1, and is in signal connection with the control module 2 for detecting an ambient temperature. The control module 2 receives the temperature input by the temperature sensor E 62, and controls the fan 61 to start to cool the interior of the housing 1. There is a fan in a general insufflator to cool it physically. However, the insufflator will be affected by external environmental factors during operating, while the fan in the conventional insufflator is always rotating, so high noise will be brought. According to the present application, the temperature of an external environment can be monitored by the temperature sensor E62. When the external ambient environment is low (lower than 20°C), the control module may receive the current temperature and control the fan to not operate, which creates a quiet environment for an indoor surgery. When the external temperature is too high, the control module will control the fan to rotate to cool the insufflator according to the temperature. In addition, whether the control module operates normally may also be determined through data comparison between the temperature sensor E 62 and the temperature sensor D52, which also provides dual guarantee for normal operation of the insufflator.

In the present embodiment, the smoke removal module 7 includes a switching valve C 71, a filter D 72, a particle detector A 73, a particle detector B 74, and a speed regulating gas pump 75. One end of the switching valve C 71 is connected to the smoke removal gas inlet 13 through the pipeline, and the other end of the switching valve C 71 is connected to the filter D 72. The other end of the filter D 72 is connected to one end of the speed regulating gas pump 75 through the pipeline. The particle detector A 73 and the particle detector B 74 are connected to the pipeline between the filter D 72 and the speed regulating gas pump 75 in sequence. Both the particle detector A 73 and the particle detector B 74 are in signal connection with the control module 2. The switching valve C 71 is in signal connection with the control module 2. The other end of the speed regulating gas pump 75 is connected to the smoke removal gas outlet 14 through the pipeline. As a more preferred solution, the particle A 73 is a PM10 detector; and the particle detector B 74is a PM2.5 detector. When a laparoscopic surgery starts, an electric knife starts to work, and the smoke in the abdominal cavity starts to be generated. First, the control module 2 controls the switching valve C71 to open, and then the speed regulating gas pump 75 is turned on. Gas starts to pass through the smoke removal module. The gas passes through the filter A 103 first to filter oil dirt, water vapor, and large particles above PM10 therein, and then the gas passes through the filter D72 which is dual filter performed to prevent the damage of the filter A 103. The treated gas passes through the particle detector A 73, that is, the PM10 detector, and the PM10 detector transmits a detected value to the control module. When the detected value is greater than a threshold value, it indicates that a front-end filter fails, and the detector may be damaged if the system continues to operate, and the system gives an alarm and enters a standby state. If the system does not give an alarm, it indicates that the system is normal. After that, the gas enters into the particle detector B 74, that is, the PM2.5 detector, and the particle detector B 74 transmits a detected value to the control module. Since the electrically cut material belongs to human tissues, and the components of the human tissues are relatively stable, it can be considered that the relative proportion of PM2.5 to PM10 in the original gas components in the abdominal cavity is constant. Therefore, the components of the original gas in the abdominal cavity can be reckoned here. A speed regulating process of the speed regulating gas pump 75 is controlled according to the detected value of the PM 2.5 detector. A comparison table of the detected value and rotating speed is stored in the control module as a preset table. The rotating speed of the speed regulating gas pump is determined according to the preset table. Automatic control can be realized by performing the above steps repeatedly. The automatic control can be stopped by only touching a key if it needs to be stopped in a special case.

In the present embodiment, a touch screen 8 is also arranged in the housing 1. The touch screen 8 is in signal connection with the control module 2 for displaying data output by various modules inside the insufflator.

In the present embodiment, the adopted control modules may be conventional single chip microcomputers or other control systems. Parts, such as various types of filters, various types of pressure sensors, various types of temperature sensors, various types of switching valves, flow sensors, safety valves, and various types of pressure relief valves are all universal standard parts or the parts known to those skilled in the art. Their structures and principles can be known through technical manuals or conventional test methods.

The use process of the present disclosure:

When the insufflator is turned on, the pneumatic module starts to operate. The external gas source is subjected to impurity removal treatment under the action of the filter C31. The treated gas directly enters into the abdominal cavity of the patient through the heating pressure relief valve 33, the safety valve 34, the proportional valve module 36, the flow sensor 37, the switching valve module 38, the insufflator gas outlet 12, and the filter 103. When a smoke removal function needs to be realized, part of the gas may enter into the smoke removal gas inlet 13 of the insufflator from the abdominal cavity of the patient through the filter 104, and finally enters into the vacuum extractor through the switching valve 71, the filter 72, the particle detector 73, the particle detector 74, the speed regulating gas pump 75, and the smoke removal gas outlet 14. Since the present equipment has high degree of automation, for an ordinary medical staff user, the equipment can be used by only regulating the preset pressure and the preset flow on the touch screen and pressing a gas supplying key after the gas paths are connected. If the gas needs to be heated/the heating needs to be turned off, a heating key only needs to be clicked on the touch screen. If the smoke in the abdominal cavity needs to be exhausted/the smoke exhaust needs to be turned off, a smoke exhausting key only needs to be clicked on the touch screen.

Various embodiments in the present specification are described in a progressive manner. Each embodiment focuses on differences from other embodiments, and the same and similar parts of various embodiments may refer to one another. The system disclosed by the embodiment is described relatively simply since it corresponds to the method disclosed by the embodiment, and relevant point may refer to the description of a method section.

Herein, specific examples are used to describe the principle and implementation manners of the present invention. The description of the embodiments above is merely intended to help understand the core idea of the constant-pressure variable-flow insufflator according to the present application.

## Claims

1. A constant-pressure variable-flow insufflator, comprising a housing (1), wherein an insufflator gas inlet (11), an insufflator gas outlet (12), a smoke removal gas inlet (13), and a smoke removal gas outlet (14) are respectively provided on an outer side of the housing (1); the insufflator gas inlet (11) is connected to an external gas source (102) through a pressure relief valve A (101); the insufflator gas outlet (12) is connectable to an abdominal cavity of a patient through a filter A (103); the smoke removal gas inlet (13) is connectable to the abdominal cavity of the patient through a filter B (104); the smoke removal gas outlet (14) is communicated with a vacuum extractor (105); a control module (2), a pneumatic module (3), a temperature detection module (4), a safety module (5), a temperature control module (6), and a smoke removal module (7) are arranged in the housing (1); the control module (2) is in signal connection with each of the pneumatic module (3), the temperature detection module (4), the safety module (5), the temperature control module (6), and the smoke removal module (7) for driving a system and performing digital signal processing on collected data; the pneumatic module (3) is configured for controlling pressure, temperature, and flow of gas delivered by the external gas source; the temperature detection module (4) is configured for detecting the temperature of the gas delivered by the external gas source; the safety module (5) is configured for detecting temperature of the control module and the pneumatic module; and the temperature control module (6) is configured for reducing noise while cooling an interior of a chassis; and the smoke removal module (7) is configured for exhausting smoke generated in the abdominal cavity of the patient,
wherein the pneumatic module (3) comprises a filter C (31), a pressure sensor A (32), a heating pressure relief valve (33), a safety valve (34), a pressure sensor B (35), a proportional valve module (36), a flow sensor (37), and a switching valve module (38); one end of the filter C (31) is connected to the insufflator gas inlet (11) through a pipeline, and another end of the filter C (31) is connected to the heating pressure relief valve (33) through the pipeline; another end of the heating pressure relief valve (33) is connected to one end of the safety valve (34) through the pipeline; another end of the safety valve (34) is connected to the proportional valve module (36) through the pipeline; one end of the proportional valve module (36) is connected to one end of the flow sensor (37) through the pipeline; another end of the flow sensor (37) is connected to one end of the switching valve module (38) through the pipeline; another end of the switching valve module (38) is connected to the insufflator gas outlet (12); the flow sensor (37) is in signal connection with the control module (2); the pressure sensor A (32) is arranged on the pipeline between the filter C (31) and the heating pressure relief valve (33); the pressure sensor A (32) is in signal connection with the control module (2); the pressure sensor B (35) is arranged on the pipeline between the safety valve (34) and the proportional valve module (36); and the pressure sensor B (35) is in signal connection with the control module (2); and
wherein the safety module (5) comprises a temperature sensor C (51) and a temperature sensor D (52); the temperature sensor C (51) is arranged on an outer side of the heating pressure relief valve (33); the temperature sensor C (51) is in signal connection with the control module (2) for detecting a temperature of the heating pressure relief valve (33); and the temperature sensor D (52) is mounted on the control module (2), and is in direct signal connection with the control module (2) for detecting a temperature of the control module (2).

2. The constant-pressure variable-flow insufflator according to claim 1, wherein the proportional valve module (36) comprises a first gas path and a second gas path connected in parallel; the first gas path and the second gas path are not opened at a same time; the first gas path consists of a pressure relief valve C (361), a pressure sensor C (362), and a proportional valve C (363); the second gas path consists of a pressure relief valve D (364), a pressure sensor D (365), and a proportional valve D (366); one end of the pressure relief valve C (361) is connected to the pressure sensor B (35) through the pipeline; another end of the pressure relief valve C (361) is connected to the proportional valve C (363) through the pipeline; another end of the proportional valve C (363) is connected to the flow sensor (37) through the pipeline; the pressure sensor C (362) is arranged on the pipeline between the pressure relief valve C (361) and the proportional valve C (363); the pressure sensor C (362) is in signal connection with the control module (2); one end of the pressure relief valve D (364) is connected to the pressure sensor B (35) through the pipeline; another end of the pressure relief valve D (364) is connected to the proportional valve D (366) through the pipeline; another end of the proportional valve D (366) is connected to the flow sensor (37) through the pipeline; the pressure sensor D (365' Is arranged on the pipeline between the pressure relief valve D (364) and the proportional valve D (366); and the pressure sensor D (365) is in signal connection with the control module (2).

3. The constant-pressure variable-flow insufflator according to claim 2, wherein the switching valve module (381) comprises a switching valve A (381), a switching valve B (382), a pressure sensor E (383), and a pressure sensor F (384); the switching valve A (381) and the switching valve B (382) are not opened or closed at a same time; one end of the switching valve A (381) is connected to the flow sensor (37), and another end of the switching valve A (381) is connected to the insufflator gas outlet (12); the pressure sensor E (383) and the pressure sensor F (384) are arranged on the pipeline between the switching valve A (381) and the insufflator gas outlet (12); the pressure sensor E (383) is connected in series with the pressure sensor F (384); and one end of the pressure sensor E(383) is connected to the switching valve B (382) through the pipeline.

4. The constant-pressure variable-flow insufflator according to claim 3, wherein the temperature detection module (4) comprises a temperature sensor A (41) and a temperature sensor B (42); the temperature sensor A (41) is arranged on the pipeline between the filter C (31) and the pressure sensor A (32); the temperature sensor A (41) is in signal connection with the control module (2); the temperature sensor B (42) is arranged on the pipeline between the pressure sensor F (384) and the insufflator gas outlet (12); and the temperature sensor B (42) is in signal connection with the control module (2).

5. The constant-pressure variable-flow insufflator according to claim 1, wherein the temperature control module (6) comprises a fan (61) and a temperature sensor E (62); the fan (61) is mounted at a bottom of the control module (2); the control module (2) is connected to the fan (61) through a circuit; the temperature sensor E (62) is mounted inside the housing (1), and is in signal connection with the control module (2) for detecting an ambient temperature; and the control module (2) receives a temperature input by the temperature sensor E (62), and controls the fan (61) to start to cool an interior of the housing (1).

6. The constant-pressure variable-flow insufflator according to claim 1, wherein the smoke removal module (7) comprises a switching valve C (71), a filter D (72), a particle detector A (73), a particle detector B (74), and a speed regulating gas pump (75); one end of the switching valve C (71) is connected to the smoke removal gas inlet (13) through the pipeline, and another end of the switching valve C (71) is connected to the filter D (72); another end of the filter D (72) is connected to one end of the speed regulating gas pump (75) through the pipeline; the particle detector A (73) and the particle detector B (74) are connected to the pipeline between the filter D (72) and the speed regulating gas pump (75) in sequence; both the particle detector A (73) and the particle detector B (74) are in signal connection with the control module (2); the switching valve C (71) is in signal connection with the control module (2); and another end of the speed regulating gas pump (75) is connected to the smoke removal gas outlet (14) through the pipeline.

7. The constant-pressure variable-flow insufflator according to claim 6, wherein the particle detector A (73) is a PM10 detector; the particle detector D (74) is a PM2.5 detector.

8. The constant-pressure variable-flow insufflator according to claim 1, wherein a touch screen (8) is also arranged in the housing (1); and the touch screen (8) is in signal connection with the control module (2) for displaying data output by various modules inside the insufflator.

## Patentansprüche

1. Insufflator mit konstantem Druck und variablem Durchfluss, umfassend ein Gehäuse (1), wobei ein Insufflator-Gaseinlass (11), ein Insufflator-Gasauslass (12), ein Rauchentfernungs-Gaseinlass (13) und ein Rauchentfernungs-Gasauslass (14) jeweils an einer Außenseite des Gehäuses (1) bereitgestellt sind; wobei der Insufflator-Gaseinlass (11) über ein Druckentlastungsventil A (101) mit einer externen Gasquelle (102) verbunden ist; wobei der Insufflator-Gasauslass (12) über einen Filter A (103) mit einer Bauchhöhle eines Patienten verbindbar ist; wobei der Rauchentfernungs-Gaseinlass (13) über einen Filter B (104) mit der Bauchhöhle des Patienten verbindbar ist; wobei der Rauchentfernungs-Gasauslass (14) mit einem Vakuumextraktor (105) kommuniziert; wobei ein Steuermodul (2), ein Pneumatikmodul (3), ein Temperturdetektionsmodul (4), ein Sicherheitsmodul (5), ein Temperatursteuermodul (6) und ein Rauchentfernungsmodul (7) in dem Gehäuse (1) angeordnet sind; wobei das Steuermodul (2) in Signalverbindung mit jedem des Pneumatikmoduls (3), des Temperaturdetektionsmoduls (4), des Sicherheitsmoduls (5), des Tempertursteuermoduls (6) und des Rauchentfernungsmoduls (7) steht, um ein System anzutreiben und eine digitale Verarbeitung von gesammelten Daten durchzuführen; wobei das Pneumatikmodul (3) ausgebildet ist, um Druck, Temperatur und Gasfluss, der von der externen Gasquelle übertragen wird, zu steuern; wobei das Temperturdetektionsmodul (4) ausgebildet ist, um die Temperatur des Gases zu detektieren, das von der externen Gasquelle übertragen wird; wobei das Sicherheitsmodul (5) ausgebildet ist, um die Temperatur des Steuermoduls und des Pneumatikmoduls zu detektieren; und wobei das Temperatursteuermodul (6) ausgebildet ist, um Geräusche zu reduzieren, während das Innere eines Chassis gekühlt wird; und wobei das Rauchentfernungsmodul (7) ausgebildet ist, um Rauch abzuführen, der in der Bauchhöhle des Patienten erzeugt wird,
wobei das Pneumatikmodul (3) einen Filter C (31), einen Drucksensor A (32), ein Heizdruckentlastungsventil (33), ein Sicherheitsventil (34), einen Drucksensor B (35), ein Proportionalventilmodul (36), einen Flusssensor (37) und ein Schaltventilmodul (38) umfasst; wobei ein Ende des Filters C (31) mit dem Insufflator-Gaseinlass (11) über eine Rohrleitung verbunden, und ein anderes Ende des Filters C (31) über die Rohrleitung mit dem Heizdruckentlastungsventil (33) verbunden ist; wobei ein anderes Ende des Heizdruckentlastungsventils (33) über die Rohrleitung mit einem Ende des Sicherheitsventils (34) verbunden ist; wobei ein anderes Ende des Sicherheitsventils (34) über die Rohrleitung mit dem Proportionalventilmodul (36) verbunden ist; wobei ein Ende des Proportionalventilmoduls (36) über die Rohrleitung mit einem Ende des Flusssensors (37) verbunden ist; wobei ein anderes Ende des Flusssensors (37) über die Rohrleitung mit einem Ende des Schaltventilmoduls (38) verbunden ist; wobei ein anderes Ende des Schaltventilmoduls (38) mit dem Insufflator-Gasauslass (12) verbunden ist; wobei der Flusssensor; wobei der Flusssensor (37) in Signalverbindung mit dem Kontrollmodul (2) steht; wobei der Drucksensor A (32) an der Rohrleitung zwischen dem Filter C (31) und dem Heizdruckentlastungsventil (33) angeordnet ist; wobei der Drucksensor A (32) in Signalverbindung mit dem Kontrollmodul (2) steht; wobei der Drucksensor B (35) an der Rohrleitung zwischen dem Sicherheitsventil (34) und dem Proportionalventilmodul (36) angeordnet ist; und wobei der Drucksensor B (35) in Signalverbindung mit dem Kontrollmodul (2) steht; und
wobei das Sicherheitsmodul (5) einen Temperatursensor C (51) und einen Temperatursensor D (52) umfasst; wobei der Temperatursensor C (51) an einer Außenseite des Heizungsdruckentlastungsventils (33) angeordnet ist; wobei der Temperatursensor C (51) in Signalverbindung mit dem Steuermodul (2) in Signalverbindung steht, um eine Temperatur des Heizungsdruckentlastungsventils (33) zu detektieren; und der Temperatursensor D (52) an dem Steuermodul (2) angebracht ist und in direkter Signalverbindung mit dem Steuermodul (2) steht, um eine Temperatur des Steuermoduls (2) zu detektieren.

2. Insufflator mit konstantem Druck und variablem Durchfluss gemäß Anspruch 1, wobei das Proportionalventilmodul (36) einen ersten Gasweg und einen zweiten Gasweg umfasst, die parallel geschaltet sind; wobei der erste Gasweg und der zweite Gasweg nicht zu einer gleichen Zeit geöffnet sind; wobei der erste Gasweg aus einem Druckentlastungsventil C (361), einem Drucksensor C (362) und einem Proportionalventil C (363) besteht; wobei der zweite Gasweg aus einem Druckentlastungsventil D (364), einem Drucksensor D (365) und einem Proportionalventil D (366) besteht; wobei ein Ende des Druckentlastungsventils C (361) über die Rohrleitung mit dem Drucksensor B (35) verbunden ist; wobei ein anderes Ende des Druckentlastungsventils C (361) über die Rohrleitung mit dem Proportionalventil C (363) verbunden ist; wobei ein anderes Ende des Proportionalventils C (363) über die Rohrleitung mit dem Flusssensor (37) verbunden ist; wobei der Drucksensor C (362) an der Rohrleitung zwischen dem Druckentlastungsventil C (361) und dem Proportionalventil C (363) angeordnet ist; wobei der Drucksensor C (362) in Signalverbindung mit dem Steuermodul (2) steht; wobei ein Ende des Druckentlastungsventils D (364) über die Rohrleitung mit dem Drucksensor B (35) verbunden ist; wobei ein anderes Ende des Druckentlastungsventils D (364) über die Rohrleitung mit dem Proportionalventil D (366) verbunden ist; wobei ein anderes Ende des Proportionalventils D (366) über die Rohrleitung mit dem Flusssensor (37) verbunden ist; wobei der Drucksensor D (365) an der Rohrleitung zwischen dem Druckentlastungsventil D (364) und dem Proportionalventil D (366) angeordnet ist; und der Drucksensor D (365) in Signalverbindung mit dem Steuermodul (2) steht.

3. Insufflator mit konstantem Druck und variablem Durchfluss gemäß Anspruch 2, wobei das Schaltventilmodul (38) ein Schaltventil A (381), ein Schaltventil B (382), einen Drucksensor E (383) und einen Drucksensor F (384) umfasst; wobei das Schaltventil A (381) und das Schaltventil B (382) nicht zu einer gleichen Zeit geöffnet oder geschlossen sind; wobei ein Ende des Schaltventils A (381) mit dem Flusssensor (37) verbunden ist und ein anderes Ende des Schaltventils A (381) mit dem Insufflator-Gasauslass (12) verbunden ist; wobei der Drucksensor E (383) und der Drucksensor F (384) an der Rohrleitung zwischen dem Schaltventil A (381) und dem Insufflator-Gasauslass (12) angeordnet sind; wobei der Drucksensor E (383) in Reihe mit dem Drucksensor F (384) geschaltet ist; und wobei ein Ende des Drucksensors E (383) über die Rohrleitung mit dem Schaltventil B (382) verbunden ist.

4. Insufflator mit konstantem Druck und variablem Durchfluss gemäß Anspruch 3, wobei das Temperturdetektionsmodul (4) einen Temperatursensor A (41) und einen Temperatursensor B (42) umfasst; wobei der Temperatursensor A (41) an der Rohrleitung zwischen dem Filter C (31) und dem Drucksensor A (32) angeordnet ist; wobei der Temperatursensor A (41) in Signalverbindung mit dem Steuermodul (2) steht; wobei der Temperatursensor B (42) an der Rohrleitung zwischen dem Drucksensor F (384) und dem Insufflator-Gasauslass (12) angeordnet ist; und wobei der Temperatursensor B (42) in Signalverbindung mit dem Steuermodul (2) steht.

5. Insufflator mit konstantem Druck und variablem Durchfluss gemäß Anspruch 1, wobei das Temperatursteuermodul (6) einen Lüfter (61) und einen Temperatursensor E (62) umfasst; wobei der Lüfter (61) an einem Boden des Steuermoduls (2) angebracht ist; wobei das Steuermodul (2) über einen Schaltkreis mit dem Lüfter (61) verbunden ist ; wobei der Temperatursensor E (62) innerhalb des Gehäuses (1) angebracht ist und in Signalverbindung mit dem Steuermodul (2) steht, um eine Umgebungstemperatur zu detektieren; und wobei das Steuermodul (2) eine Temperatureingabe durch den Temperatursensor E (62) empfängt und den Lüfter (61) so steuert, dass er beginnt, einen Innenraum des Gehäuses (1) zu kühlen.

6. Insufflator mit konstantem Druck und variablem Durchfluss gemäß Anspruch 1, wobei das Rauchentfernungsmodul (7) ein Schaltventil C (71), einen Filter D (72), einen Partikeldetektor A (73), einen Partikeldetektor B (74) und eine geschwindigkeitsregulierende Gaspumpe (75) umfasst; wobei ein Ende des Schaltventils C (71) über die Rohrleitung mit dem Rauchentfernungs-Gaseinlass (13) verbunden ist, und ein anderes Ende des Schaltventils C (71) mit dem Filter D (72) verbunden ist; wobei ein anderes Ende des Filters D (72) über die Rohrleitung mit einem Ende der geschwindigkeitsregulierenden Gaspumpe (75) verbunden ist; wobei der Partikeldetektor A (73) und der Partikeldetektor B (74) mit der Rohrleitung zwischen dem Filter D (72) und der geschwindigkeitsregulierenden Gaspumpe (75) in Reihe verbunden sind; wobei sowohl der Partikeldetektor A (73) als auch der Partikeldetektor B (74) in Signalverbindung mit dem Steuermodul (2) stehen; wobei das Schaltventil C (71) in Signalverbindung mit dem Steuermodul (2) steht; und wobei ein anderes Ende der geschwindigkeitsregulierenden Gaspumpe (75) über die Rohrleitung mit dem Rauchentfernungsgasauslass (14) verbunden ist.

7. Insufflator mit konstantem Druck und variablem Durchfluss gemäß Anspruch 6, wobei der Partikeldetektor A (73) ein PM10-Detektor ist; wobei der Partikeldetektor D (74) ein PM2,5-Detektor ist.

8. Insufflator mit konstantem Druck und variablem Durchfluss gemäß Anspruch 1, wobei ein Touchscreen (8) ebenfalls in dem Gehäuse (1) angeordnet ist; und wobei der Touchscreen (8) in Signalverbindung mit dem Steuermodul (2) steht, um Daten anzuzeigen, die von verschiedenen Modulen innerhalb des Insufflators ausgegeben werden.

## Revendications

1. Insufflateur à flux variable et pression constante, comprenant un logement (1), dans lequel un orifice d'entrée de gaz d'insufflateur (11), un orifice de sortie de gaz d'insufflateur (12), un orifice d'entrée de gaz d'extraction de fumée (13), et un orifice de sortie de gaz d'extraction de fumée (14) sont respectivement disposés sur un côté externe du logement (1) ; l'orifice d'entrée de gaz d'insufflateur (11) est raccordé à une source de gaz externe (102) à travers une soupape de décompression A (101) ; l'orifice de sortie de gaz d'insufflateur (12) pouvant être raccordé à une cavité abdominale d'un patient à travers un filtre A (103) ; l'orifice d'entrée de gaz d'extraction de fumée (13) pouvant être raccordé à la cavité abdominale du patient à travers un filtre B (104) ; l'orifice de sortie de gaz d'extraction de fumée (14) communique avec un extracteur de vide (105) ; un module de commande (2), un module pneumatique (3), un module de détection de température (4), un module de sécurité (5), un module de commande de température (6), et un module d'extraction de fumée (7) sont agencés dans le logement (1) ; le module de commande (2) se trouve en connexion de signal avec chacun du module pneumatique (3), du module de détection de température (4), du module de sécurité (5), du module de commande de température (6), et du module d'extraction de fumée (7) pour piloter un système et effectuer le traitement de signal numérique sur les données recueillies ; le module pneumatique (3) est configuré pour commander la pression, la température, et le flux de gaz délivré par la source de gaz externe ; le module de détection de température (4) est configuré pour détecter la température du gaz délivré par la source de gaz externe ; le module de sécurité (5) est configuré pour détecter la température du module de commande et du module pneumatique ; et le module de commande de température (6) est configuré pour réduire le bruit tout en refroidissant un intérieur d'un châssis ; et le module d'extraction de fumée (7) est configuré pour faire sortir la fumée générée dans la cavité abdominale du patient,
dans lequel le module pneumatique (3) comprend un filtre C (31), un capteur de pression A (32), une soupape de décompression chauffante (33), une soupape de sécurité (34), un capteur de pression B (35), un module à soupape proportionnelle (36), un capteur de flux (37), et un module valve de commutation (38) ; une extrémité du filtre C (31) est raccordée à l'orifice d'entrée de gaz d'insufflateur (11) à travers une conduite, et une autre extrémité du filtre C (31) est raccordée à la soupape de décompression chauffante (33) à travers la conduite ; une autre extrémité de la soupape de décompression chauffante (33) est raccordée à une extrémité de la soupape de sécurité (34) à travers la conduite ; une autre extrémité de la soupape de sécurité (34) est raccordée au module à soupape proportionnelle (36) à travers la conduite ; une extrémité du module à soupape proportionnelle (36) est raccordée à une extrémité du capteur de flux (37) à travers la conduite ; une autre extrémité du capteur de flux (37) est raccordée à une extrémité du module valve de commutation (38) à travers la conduite ; une autre extrémité du module valve de commutation (38) est raccordée à l'orifice de sortie de gaz d'insufflateur (12) ; le capteur de flux (37) se trouve en connexion de signal avec le module de commande (2) ; le capteur de pression A (32) est agencé sur la conduite entre le filtre C (31) et la soupape de décompression chauffante (33) ; le capteur de pression A (32) se trouve en connexion de signal avec le module de commande (2) ; le capteur de pression B (35) est agencé sur la conduite entre la soupape de sécurité (34) et le module à soupape proportionnelle (36) ; et le capteur de pression B (35) se trouve en connexion de signal avec le module de commande (2) ; et
dans lequel le module de sécurité (5) comprend un capteur de température C (51) et un capteur de température D (52) ; le capteur de température C (51) est agencé sur un côté externe de la soupape de décompression chauffante (33) ; le capteur de température C (51) se trouve en connexion de signal avec le module de commande (2) pour détecter une température de la soupape de décompression chauffante (33) ; et le capteur de température D (52) est monté sur le module de commande (2), et se trouve en connexion directe de signal avec le module de commande (2) pour détecter une température du module de commande (2).

2. Insufflateur à flux variable et pression constante selon la revendication 1, dans lequel le module à soupape proportionnelle (36) comprend un premier trajet de gaz et un second trajet de gaz connectés en parallèle ; le premier trajet de gaz et le second trajet de gaz ne sont pas ouverts en même temps ; le premier trajet de gaz est constitué d'une soupape de décompression C (361), d'un capteur de pression C (362), et d'une valve proportionnelle C (363) ; le second trajet de gaz est constitué d'une soupape de décompression D (364), d'un capteur de pression D (365), et d'une valve proportionnelle D (366) ; une extrémité de la soupape de décompression C (361) est raccordée au capteur de pression B (35) à travers la conduite ; une autre extrémité de la soupape de décompression C (361) est raccordée à la valve proportionnelle C (363) à travers la conduite ; une autre extrémité de la valve proportionnelle C (363) est raccordée au capteur de flux (37) à travers la conduite ; le capteur de pression C (362) est agencé sur la conduite entre la soupape de décompression C (361) et la valve proportionnelle C (363) ; le capteur de pression C (362) se trouve en connexion de signal avec le module de commande (2) ; une extrémité de la soupape de décompression D (364) est raccordée au capteur de pression B (35) à travers la conduite ; une autre extrémité de la soupape de décompression D (364) est raccordée à la valve proportionnelle D (366) à travers la conduite ; une autre extrémité de la valve proportionnelle D (366) est raccordée au capteur de flux (37) à travers la conduite ; le capteur de pression D (365) est agencé sur la conduite entre la soupape de décompression D (364) et la valve proportionnelle D (366) ; et le capteur de pression D (365) se trouve en connexion de signal avec le module de commande (2).

3. Insufflateur à flux variable et pression constante selon la revendication 2, dans lequel le module valve de commutation (38) comprend une valve de commutation A (381), une valve de commutation B (382), un capteur de pression E (383), et un capteur de pression F (384) ; la valve de commutation A (381) et la valve de commutation B (382) ne sont pas ouvertes ou fermées en même temps ; une extrémité de la valve de commutation A (381) est raccordée au capteur de flux (37), et une autre extrémité de la valve de commutation A (381) est raccordée à l'orifice de sortie de gaz d'insufflateur (12) ; le capteur de pression E (383) et le capteur de pression F (384) sont agencés sur la conduite entre la valve de commutation A (381) et l'orifice de sortie de gaz d'insufflateur (12) ; le capteur de pression E (383) est raccordé en série au capteur de pression F (384) ; et une extrémité du capteur de pression E (383) est raccordée à la valve de commutation B (382) à travers la conduite.

4. Insufflateur à flux variable et pression constante selon la revendication 3, dans lequel le module de détection de température (4) comprend un capteur de température A (41) et un capteur de température B (42) ; le capteur de température A (41) est agencé sur la conduite entre le filtre C (31) et le capteur de pression A (32) ; le capteur de température A (41) se trouve en connexion de signal avec le module de commande (2) ; le capteur de température B (42) est agencé sur la conduite entre le capteur de pression F (384) et l'orifice de sortie de gaz d'insufflateur (12) ; et le capteur de température B (42) se trouve en connexion de signal avec le module de commande (2).

5. Insufflateur à flux variable et pression constante selon la revendication 1, dans lequel le module de commande de température (6) comprend un ventilateur (61) et un capteur de température E (62) ; le ventilateur (61) est monté au niveau d'un fond du module de commande (2) ; le module de commande (2) est raccordé au ventilateur (61) à travers un circuit ; le capteur de température E (62) est monté à l'intérieur du logement (1), et se trouve en connexion de signal avec le module de commande (2) pour détecter une température ambiante ; et le module de commande (2) reçoit une entrée de température par le capteur de température E (62), et commande au ventilateur (61) de commencer à refroidir un intérieur du logement (1).

6. Insufflateur à flux variable et pression constante selon la revendication 1, dans lequel le module d'extraction de fumée (7) comprend une valve de commutation C (71), un filtre D (72), un détecteur de particules A (73), un détecteur de particules B (74), et une pompe à gaz de régulation de vitesse (75) ; une extrémité de la valve de commutation C (71) est raccordée à l'orifice d'entrée de gaz d'extraction de fumée (13) à travers la conduite, et une autre extrémité de la valve de commutation C (71) est raccordée au filtre D (72) ; une autre extrémité du filtre D (72) est raccordée à une extrémité de la pompe à gaz de régulation de vitesse (75) à travers la conduite ; le détecteur de particules A (73) et le détecteur de particules B (74) sont raccordés à la conduite entre le filtre D (72) et la pompe à gaz de régulation de vitesse (75) en séquence ; à la fois le détecteur de particules A (73) et le détecteur de particules B (74) se trouvent en connexion de signal avec le module de commande (2) ; la valve de commutation C (71) se trouve en connexion de signal avec le module de commande (2) ; et une autre extrémité de la pompe à gaz de régulation de vitesse (75) est raccordée à l'orifice de sortie de gaz d'extraction de fumée (14) à travers la conduite.

7. Insufflateur à flux variable et pression constante selon la revendication 6, dans lequel le détecteur de particules A (73) est un détecteur PM10 ; le détecteur de particules D (74) est un détecteur PM2.5.

8. Insufflateur à flux variable et pression constante selon la revendication 1, dans lequel un écran tactile (8) est également agencé dans le logement (1) ; et l'écran tactile (8) se trouve en connexion de signal avec le module de commande (2) pour afficher la sortie des données à travers divers modules à l'intérieur de l'insufflateur.
